Europäisches Patentamt

European Patent Office

Office européen des brevets

(19)

(11) Publication number: **0 107 476**
A2

# EUROPEAN PATENT APPLICATION

(21) Application number: 83306329.0

(22) Date of filing: 18.10.83

(51) Int. Cl.³: **A 61 F 1/00**, A 61 B 17/18

(30) Priority: 25.10.82 US 436269

(43) Date of publication of application: 02.05.84
Bulletin 84/18

(84) Designated Contracting States: **AT BE CH DE FR GB IT LI LU NL SE**

(71) Applicant: **Calcitek, Inc., 4125-B Sorrento Valley Boulevard, San Diego California 92121 (US)**

(72) Inventor: **Kay, John F., 445 Delage Drive, Encinitas California, 92024 (US)**
Inventor: **Comstock, Donald M., 8220 Vincetta Drive, La Mesa California, 92041 (US)**

(74) Representative: **Lawrence, Malcolm Graham et al, BROOKES & MARTIN High Holborn House 52/54 High Holborn, London WC1V 6SE (GB)**

(54) **Bone graft substitute.**

(57) A bone graft has a dense calcium-phosphate ceramic core and porous calcium-phosphate ceramic surface layers for directly obtaining viable bone-growth surfaces. The graft is implanted as a replacement or extension segment for a natural bone segment or to repair a deformity with its porous end layers in intimate contact with osseous material at prepared ends of the bone. The dense core provides the required flexural and compressive strength whereas the porous surface layers promote bone ingrowth that fuses the bone to the graft.

EP 0 107 476 A2

-1-

## BONE GRAFT SUBSTITUTE

The present invention relates to bone graft substitutes and more particularly to bone graft substitutes formed of calcium-phosphates, such as hydroxylapatite or whitlockite.

### BACKGROUND OF THE INVENTION

There exists a need for improved grafts to replace portions of natural bones. In certain accidents, bone is so badly crushed or splintered that conventional setting coupled with natural healing processes will not repair the bone to the extent that sufficient strength for normal activity is restored. A crushed jaw bone, for example, will frequently be irreparable by the normal setting and healing process. In other instances, portions of bone must be surgically removed, e.g., to correct bone deformities or to excise local bone cancers.

It has long been recognized that hydroxylapatite, $Ca_{10}(PO_4)_6(OH)_2$ and whitlockite, $\beta-Ca_3(PO_4)_2$, ceramics have properties which make them particularly suitable for use in bone or dental prostheses. Hydroxylapatite is the major mineral phase of both tooth and bone and therefore fully compatible with bone and other tissue. It is a non-resorbable ceramic material which in dense, porous, or particulate form provides continual restorative or corrective performance. Whitlockite, in contrast, is bio-resorbable, the rate of dissolution being proportional to its surface area, i.e., porous and particulate forms resorb much faster than dense block sections. Attempts to provide suitable hydroxylapatite and whitlockite bulk bone grafts or dental prostheses, however, have heretofore been less than successful due to the difficulty of forming hydroxylapatite or whitlockite grafts of sufficient strength which will rapidly fuse with the bone during initial healing.

-2-

A bone graft substitute must have both tensile and compressive strength sufficient to withstand the forces it experiences in the site of the natural bone portion it replaces and should also provide an interface with bone tissue that promotes rapid attachment of bone tissue to achieve integration of the remaining bone and the bone graft. Inserts of hydroxylapatite have previously been produced with sufficient porosity to promote bone ingrowth, but the required porosity has been achieved at the expense of adequate compressive strength for most prosthetic applications. The inadequacy of certain prior attempts to produce suitable hydroxylapatite prostheses are detailed in U.S. Patent No. 4,097,935. Porous whitlockite suffers from the same low strength characteristics that degrade even further as the material is resorbed.

Patent No. 4,097,935 teaches that, opposed to producing prosthetic hydroxylapatite ceramics by sintering powdered hydroxylapatite, as had been attempted previously, prosthetic ceramics may be produced by sintering a gelatinous precipitate of calcium and phosphate. The ceramics are primarily hydroxylapatite but may contain some whitlockite, ß-tricalcium phosphate, and other calcium-phosphate compounds, depending on the calcium to phosphate ratio. Generally, when the gelatinous precipitate is sintered, a solid, non-porous, ceramic is formed. However, if the precipitate contains particulate organic material, such as powdered starch, cellulose, cotton or collagen, in amounts ranging from 5 to 25 weight percent of the calcium plus phosphate, the ceramic will have substantial porosity.

Providing that a sufficiently strong calcium-phosphate ceramic can be formed by sintering powdered material, the method of sintering powder has an advantage over methods of forming ceramic material from

-3-

gelatinous calcium-phosphate precipitates or other precursors with high water content. The compacted calcium-phosphate powders are formed directly to shape during the pressing step, experiencing shrinkage only as densification occurs during sintering. The gelations materials, however, experience significant shrinkage as the water is removed and a "green body" is formed, and further shrinkage occurs as densification by sintening takes place. The latter process allows for a greater chance of flaw development due to nonuniform shrinkage or varying cross-sectional areas.

In U.S. Patent No. 3,855,638, a porous metal surface layer is applied to a solid metal substrate by sintering a slurry of metallic powder and organic binders onto the previously-formed substrate. The organic binders burn off during sintering leaving pores in the metallic surface layer. While such a procedure might work with metals, a hydroxylapatite or whitlockite ceramic prosthetic device could not be formed by sintering a mixture of hydroxylapatite or whitlockite powder and organic materials to a solid sintered ceramic substrate, because such a procedure would form a device with a weak interface between the porous ceramic layer and the dense substrate, causing the porous ceramic layer to crack off the dense substrate when subjected to significant stress.

## SUMMARY OF THE INVENTION

A prosthetic ceramic material is formed having a solid, non-porous hydroxylapatite or whitlockite core and integrally formed porous hydroxylapatite or whitlockite surface layers with continuous interfaces between the core and the surface layers that resist interfacial failure. A structure is molded by layering powdered material in a mold, the layers which are to form the porous surface layers comprising a mixture of hydroxylapatite and/or whitlockite powder and powdered

0107476

-4-

organic material and the layer which is to form the non-porous core comprising substantially pure hydroxylapatite and/or whitlockite powder. The layers are tamped down as they are laid, and after the mold is filled, the layered powders are compacted in a molding press to about 55-175MPa pressure and then heated to between 1050°C and about 1300°C for a period sufficient to sinter the green compacted body into a fused structure. During this heating process, the organic material is volatilized, leaving pores in the surface layers. The core has substantial tensile and compressive strength, whereas the pores of the surface layers provide for bone ingrowth and rapid initial stabilization. After ingrowth has taken place, the bone with such a fully integrated graft is again strong.

BRIEF DESCRIPTION OF THE DRAWINGS

FIGURE 1 is a cross-sectional view of a mold in which powdered material is layered according to the present invention;

FIGURE 2 is perspective view of a bone graft (in this case for a genioplasty) embodying various features of the invention;

FIGURE 3a is a side elevation view of a mandible, partially cut away, which has been grafted with the bone graft of FIGURE 2;

FIGURE 3b is a front elevation view of the grafted mandible of FIGURE 3a; and

FIGURE 4 is a block diagram representing steps of a process, embodying various features of the invention, by which a bone graft is formed.

DETAILED DESCRIPTION OF THE PREFERRED EMBODIMENT

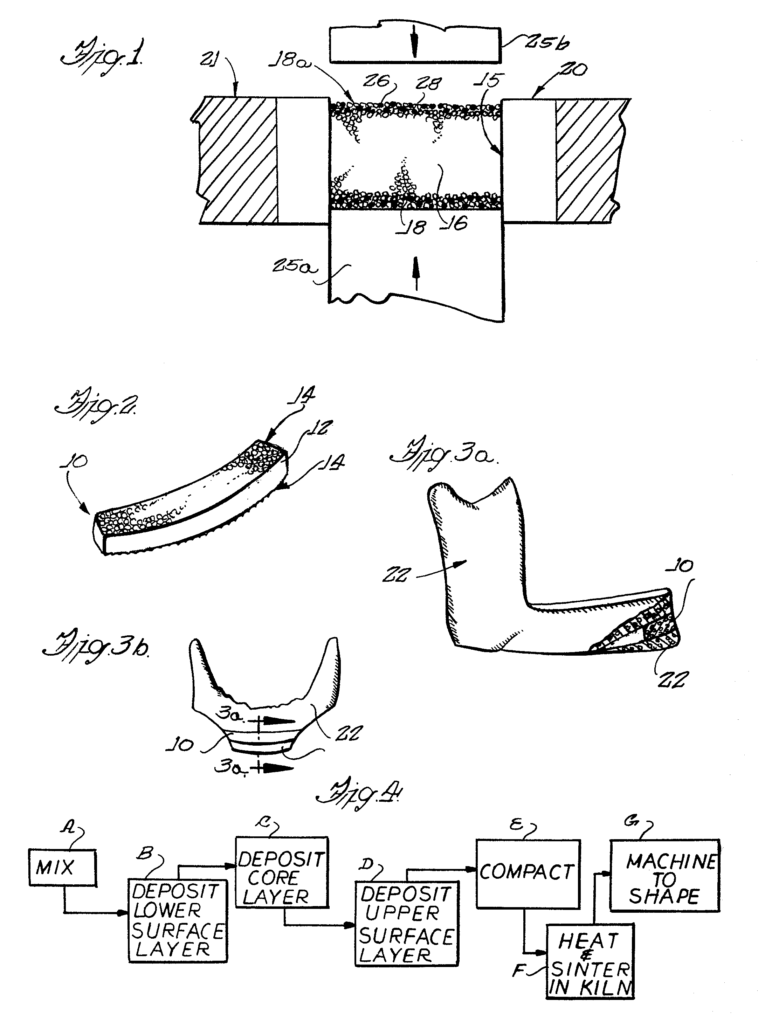

In accordance with the present invention, a bone graft 10 (FIG. 2), formed either entirely or primarily of calcium phosphate ceramic material, such as hydroxylapatite and/or whitlockite, has a strong, dense, non-porous core 12 and integrally joined porous hydroxylapatite and/or whitlockite surface layers 14 that interface with natural bone (osseous) material and into which bone material grows. The bone graft 10 is formed by sintering a green graft of powdered material that has been deposited as layers in a cavity 15 of a mold or die 20 (FIG. 1) and compacted at high pressures. The core 12 is formed from a central layer 16 (FIG. 1) of substantially pure hydroxylapatite and/or whitlockite powder, whereas the porous surface layers 14 are formed from layers 18 of hydroxylapatite and/or whitlockite powder mixed with powdered organic material that is volatilized and disappears during sintering.

Calcium-phosphate ceramic will be used herein to refer to hydroxylapatite, whitlockite and mixtures thereof. Generally a graft 10 will be formed of hydroxylapatite where permanecy and substantial strength are required, whereas a graft will be formed of whitlockite where it is desired that the graft be resorbed and eventually replaced by bone tissue. In certain applications, whitlockite and hydroxylapatite may be mixed to form a semi-resorbable prosthesis. Different portions of the graft 10 may be formed of different calcium-phosphate material; for example, the dense core 12 may be formed of hydroxylapatite while the porous surface layers 14 are formed of resorbable whitlockite.

The green graft is formed in a mold 20, such as that illustrated in FIGURE 1 including a supporting die table 21, having a cavity 15 with a configuration similar to the portion of the bone 22 which the graft is intended to replace. Because shrinkage does occur as the calcium-phosphate ceramic powder fuses into an integral unit, the mold is typically somewhat larger than the graft which will be produced. The mold 20 shown in FIGURE 1 is configured for forming the generally flat, arcuate graft that is shown in FIGURE 2 and intended for forming a bone graft that is used to extend the mental process of the mandible (genioplasty) (FIGS. 3a, 3b). This graft 10 has a flat core 12 and two spaced-apart, generally parallel end surface layers 14. A graft of non-parallel surface layers is also possible by modification of the compaction tooling. In some cases, it may be necessary to machine the sintered graft 10 to its final shape matching the configuration of an excised bone segment; alternatively, the material may be made in bulk and simply cut to shape.

The mold 20 is installed in a press, typically hydraulic in nature, whose compaction pressure is adjustable by changing the oil pressure to hydraulic cylinders (not shown). A lower piston-driven punch 25a is moved into the lower end of the cavity 15, and the lower layer 18, the core layer 16 and the upper 18a deposited thereupon. Then an upper piston-driven punch 25b is moved downward into the die cavity 15 to apply compacting pressure to the layers. A mechanical, electrical or hand operated press (or clamp frame) may also be used to compact the green body.

It is desirable to use a calcium-phosphate ceramic powder in as pure a form as possible in order to maximize the strength and homogeneity of the bone graft. For purposes of the invention, the calcium-phosphate powder is preferably at least about 97 weight percent pure and most preferably at least about 99 weight percent pure calcium-phosphate in hydroxylapatite and/or whitlockite form.

Small particle size processed calcium-phosphate powders are found to produce the best results as they can be closely packed with minimum interstitial volume, and for purposes of this invention, it is preferred that the calcium-phosphate powder be able to sift through a screen having openings less than 175 microns (80 mesh).

-7-

The amount of powdered organic material (represented as solid circles 26 in FIG. 1) which is intermixed with the ceramic (hydroxylapatite and/or whitlockite) powder (represented as clear circles 28) depends on the desired porosity of the surface layer 14. Generally the organic material particles 26 comprise between about 10 volume percent and about 60 volume percent of the powder mixture that forms the surface layer, and the ceramic material particles 28 comprise the remaining 40-90 volume percent. Typically the organic material particles comprise about 50 volume percent of this mixture.

It is preferred that the particle size of the organic material be chosen such that resultant surface porosity falls generally in the 100-250 micron range, and for purposes of this invention, the particle size of the organic material is generally such that the particles of organic material will sift through a screen having square openings with dimensions between about two and about seven times those of a screen through which the ceramic powder sifts.

The choice of the particulate organic material that is mixed with the ceramic powder to form the surface layers is not considered to be particularly critical so long as the organic material is volatilized substantially without trace at or below the sintering temperatures, i.e., at or below 1050°C. Very suitable organic materials for providing the pores are polyvinyl alcohol (PVA), polyethyleneglycol (PEG), or urea. Other suitable materials include powdered starch, cellulose, cotton or collagen. The shape of the pore-forming organic material is chosen to be consistant with that of the final pore geometry desired.

With reference to the block diagram of FIG. 4, the organic material particles are blended (A) in a mixer with hydroxylapatite and/or whitlockite particles.  The resulting homogeneous mixture is then deposited (B) as a lower surface layer 18 in the mold cavity 15 to a depth of between about 0.5 and about 8.0 mm, i.e., a depth sufficient to form a fused surface layer with a thickness of between about 0.25 and about 4.0 mm, and tamped down with a flat-headed tool (not shown) sufficiently so that relatively little mixing occurs when the core-forming layer of calcium-phosphate ceramic powder is deposited thereon.  Of course, slight mixing will occur at the interface when the layers are later pressed against each other, but this is considered advantageous because it provides an interfacial region that blends somewhat gradually from porous to non-porous.  Calcium-phosphate ceramic powder is deposited (C) as a second, deep, homogeneous layer 16 on top of the first mixed layer 18 to a depth a depth of between about 5 and about 80 mm, sufficient to provide a bone graft core of desired thickness, usually about 2.5 to 40 mm., and this layer is similarly tamped down. Next a third upper layer 18, generally equal in composition and thickness to the first layer, is deposited (D) and tamped down.  Then the entire layered powder structure is compacted (E), e.g., by activating the hydraulic cylinders to press the die punches 25a,b against the mixed layers, applying between about 55 and 175 MPa compaction pressure.  The green compact is then removed from the die.

The green compact is placed in a kiln (F) and heated slowly to a temperature at which the pore forming organic material is gradually volatilized, typically about 100°C to about 275°C.  The temperature is then raised over a period of several hours to between about 1050°C and about 1300°C, preferably between about 1050°C and about 1175°C, for a period of time until the calcium-phosphate ceramic powder in all layers has fused.  The fusing time depends on the dimensions of the mold, but will typically be between about 10 and about 200 minutes at temperatures above 1050°C. The dense central layer 16 of the green compact fuses into a solid, non-porous core. The ceramic particles 28 in the mixed layers 18 fuse together in the spatial arrangement which includes the voids left by the volatilized organic particles 26, whereby the surface layers 14 have substantial porosity. The sintered porous layers 14 are commonly covered with a thin, friable surface coating of sintered ceramic with significantly fewer exposed pores than are present just below this layer. This coating results from the compressive action of the faces of the punches 25 during compaction, and is easily scraped or machined away, leaving the desired porous surface texture.

Although some shrinkage of the structure occurs during sintering as the sintering ceramic powder fills interstitial voids of the compressed powder, shrinkage is typically less than about 50 volume percent and less than about 28 percent in any dimension. Because of this relatively small amount of shrinkage overall, differential shrinkage between the core layer and surface layers is negligable. There is no discontinuity at the interfaces between the porous and non-porous layers, and a faultless bone graft is formed from which the porous surface layers will not spall or fragment after implantation.

If the ceramic powder used is substantially pure hydroxylapatite, the dense solid hydroxylapatite core 12 has a density of at least 3.05g/cm$^3$, i.e., at least about 97 percent of the maximum theoretical density of hydroxylapatite. Its flexural strength, as measured by the modulus of rupture test, ASTM C369-56, is above about 137MN/m$^2$, and its compressive strength is above about 550MN/m$^2$ as measured by the compressive strength test, ASTM C407-58; its Knoop hardness is above about 450.

If the ceramic powder used is substantially pure whitlockite, the dense solid whitlockite core has a density of at least $2.91g/cm^3$, i.e., at least about 97% of the maximum theoretical density of whitlockite. Its flexural strength, as measured by the modulus of rupture test (ASTM C369-56), is above $137MN/m^2$, and its compressive strength is above $515MN/m^2$ as measured by the compressive strength test, ASTM C407-58; its Knoop hardness is above 450.

The compressive and flexural strength of the surface layers vary depending on their porosity, but are lower than that of the bulk, dense core.

The strength of the ceramic bone graft approximates that of bone, however, due to its organic matrix, bone is much more compliant. The ceramic bone graft substitutes are designed to repair or replace bone segments that would experience predominantly compressive forces. While the porous surface layers 14 are not initially comparable in strength to the bone 22, after bone ingrowth has occurred fusing the bone and graft 10 into a unitary structure, the surface layers will provide a smooth transition in strength from the bone to the graft core 12.

The strength of the grafts is such that, in the present state of development, the grafts formed in accordance with the invention are most generally suitable for replacing relatively small bone segements, e.g., having maximum cross-sectional dimensions of about 40 mm, in areas where predominantly compressive forces are experienced. Replacements for long bone segments will generally have solid cores between about 5 and about 40 mm. in thickness. Larger grafts may be used for replacing bone segments which are not generally subjected to substantial tensile stress.

-11-

After the bone graft is sintered, it may be machined (G) along its sides (to any extent) to more closely approximate the shape of the bone segment it is to replace, however it may only be machined on its porous surfaces such that a porous layer at least 100 microns thick remains to facilitate tissue ingrowth and initial stabilization upon implantation.

For implantation, the bone is usually cut with cutting tools used routinely in orthopedic or oral/maxillfacial surgical practice or with diamond burs, and the bone graft 10 and the bone graft is inserted with its porous surface layers 14 in intimate contact with osseous tissue at the prepared ends of the bone. The bone is immobilized in a conventional manner during a healing period in which osseous tissue grows into the pores of the surface layers and calcification occurs at the bone-graft interfaces.

EXAMPLE 1

Powdered hydroxylapatite (97 percent purity) is prepared using conventional synthetic procedures. The hydroxylapatite is sifted through a screen having 150 micron square openings. A portion of this hydroxylapatite is mixed in equal volume with PEG spheres having an average diameter of 700 microns.

In a round mold 20, having a 40 mm diameter cavity 15, a 1.0 mm thick layer 18 of the hydroxylapatite/PEG mixture is deposited, and this mixture is tamped down. Hydroxylapetite powder is deposited in a 20 mm thick second layer 16 and tamped down, and a 1.0 mm thick third layer 18 of hydroxylapatite/PEG mixture is deposited and tamped. The die punches 25 are closed against the layered composition initially applying 100 MPa pressure thereto. The compacted part is ejected and placed in kiln and slowly heated to 270°C and held at this temperature for 2 hours; the temperature is then slowly

0107476

-12-

raised. After the temperature has reached 1120°C, the contents are cooled to room temperature over a period of about 6 hours and then removed from the kiln.

### EXAMPLE 2

Powdered whitlockite (97 percent purity) is prepared using conventional synthetic procedures. The whitlockite is sifted through a screen having 150 micron square openings. A portion of this whitlockite is mixed in equal volume with PVA spheres having an average diameter of 700 microns.

In a round mold 20, having a 40 mm diameter cavity 15, a 1.0 mm thick layer 18 of the whitlockite/PVA mixture is deposited, and this mixture is tamped down. Whitlockite powder is deposited in a 20 mm thick second layer 16 and tamped down, and a 1.0 mm thick third layer 18 of whitlockite/PVA mixture is deposited and tamped. The die punches are closed against the layered composition initially applying 100 MPa pressure thereto. The compacted part is ejected and placed in a kiln and slowly heated to 270°C and held for 2 hours; the temperature is then slowly raised. After the temperature has reached 1250°C, the contents are allowed to remain in the kiln as the kiln is cooled to room temperature over a period of about 6 hours and then removed from the kiln.

### EXAMPLE 3

Powdered whitlockite (97 percent purity) is prepared using conventional synthetic procedures. The whitlockite is sifted through a screen having 150 micron square openings. A portion of this whitlockite is mixed in equal volume with urea spheres having an average diameter of 700 microns.

In a round mold 20, having a 40 mm diameter cavity 15, a 1.0 mm thick layer 18 of the whitlockite/urea mixture is deposited, and this mixture is tamped down. Whitlockite powder is deposited in a 20

-13-

mm thick second layer 16 and tamped down, and a 1.0 mm. thick third layer 18 of whitlockite/urea mixture is deposited and tamped. The die punches are closed against the layered composition initially applying 100 MPa pressure thereto. The compacted part is ejected and placed in a kiln and slowly heated to 270°C and held for 2 hours; the temperature is then slowly raised. After the temperature has reached 1250°C, the contents are allowed to remain in the kiln as the kiln is cooled to room temperature over a period of about 6 hours and then removed from the kiln.

### EXAMPLE 4

Powdered hydroxylapatite (97 percent purity) and powdered whitlockite (97 percent purity) are prepared using conventional synthetic procedures. The powders are each sifted through a screen having 150 micron square openings. The whitlockite is mixed in equal volume with urea spheres having an average diameter of 700 microns.

In a round mold 20, having a 40 mm diameter cavity 15, a 1.0 mm thick layer 18 of the whitlockite/urea mixture is deposited, and this mixture is tamped down. Hydroxylapatite powder is deposited in a 20 mm thick second layer 16 and tamped down, and a 1.0 mm thick third layer 18 of whitlockite/urea mixture is deposited and tamped. The die punches are closed against the layered composition initially applying 100 MPa pressure thereto. The compacted part is ejected and placed in a kiln and slowly heated to 270°C and held for 2 hours; the temperature is then slowly raised. After the temperature has reached 1250°C, the contents are allowed to remain in the kiln as the kiln is cooled to room temperature over a period of about 6 hours and then removed from the kiln.

### EXAMPLE 5

Powdered hydroxylapatite (97 percent purity)

-14-

amd powdered whitlockite (97 percent purity) are prepared using conventional synthetic procedures. The powders are each sifted through a screen having 150 micron square openings. A portion of the whitlockite is mixed uniformly with the hydroxylapatite in a whitlockite to hydroxylapatite weight ratio of about 1 to 1. A portion of the whitlockite is mixed in equal volume with PVA spheres having an average diameter of 700 microns.

In a round mold 20, having a 40 mm diameter cavity 15, a 1.0 mm thick layer 18 of the whitlockite/PVA mixture is deposited, and this mixture is tamped down. Whitlockite/hydroxylapatite powder is deposited in a 20 mm thick second layer 16 and tamped down, and a 1.0 mm. thick third layer 18 of whitlockite/PVA mixture is deposited and tamped. The die punches are closed against the layered composition initially applying 100 MPa pressure thereto. The compacted part is ejected and placed in a kiln and slowly heated to 270°C and held for 2 hours; the temperature is then slowly raised. After the temperature has reached 1250°C, the contents are allowed to remain in the kiln as the kiln is cooled to room temperature over a period of about 6 hours and then removed from the kiln.

## EXAMPLE 6

Powdered hydroxylapatite (97 percent purity) amd powdered whitlockite (97 percent purity) are prepared using conventional synthetic procedures. The powders are each sifted through a screen having 150 micron square openings. A portion of the whitlockite/hydroxylapatite is mixed uniformly with the hydroxylapatite in a whitlockite to hydroxylapatite weight ratio of about 1 to 1. The whitlockite/ hydroxylapatite mixture is mixed in equal volume with PEG spheres having an average diameter of 700 microns.

-15-

In a round mold 20, having a 40 mm diameter cavity 15, a 1.0 mm thick layer 18 of the whitlockite/hydroxylapatite/PEG mixture is deposited, and this mixture is tamped down. Hydroxylapatite powder is deposited in a 20 mm thick second layer 16 and tamped down, and a 1.0 mm. thick third layer 18 of whitlockite/hydroxylapatite/PEG mixture is deposited and tamped. The die punches are closed against the layered composition initially applying 100 MPa pressure thereto. The compacted part is ejected and placed in a kiln and slowly heated to 270°C and held for 2 hours; the temperature is then slowly raised. After the temperature has reached 1250°C, the contents remains in the kiln as the kiln and its contents are cooled to room temperature over a period of about 6 hours and then removed from the kiln.

While the invention has been described in terms of certain preferred embodiments, modifications obvious to one with ordinary skill in the art might be made without departing from the scope of the invention. For example, the porous surfaces need not be limited to end surfaces, and to the extent that a thin packed layer can be formed within a contoured mold, an organic material surface layer may be packed within the mold and the ceramic powder hydroxylapatite powder packed within a pocket created by a packed, contoured surface layer. An implant for an end of a bone, such as a finger tip, would be formed with a single porous surface layer. For long bone replacement, a composite structure might be strengthened by incorporating a reinforcing framework, e.g., one formed of metal wire, within the packed hydroxylapatite or whitlockite layer.

Various features of the invention are emphasized in the following claims.

CLAIMS

1. A bone graft comprising

a solid non-porous ceramic core of substantially pure calcium-phosphate ceramic material, the core having a flexural strength of at least about $137MN/m^2$ and a compressive strength of at least $550MN/m^2$, and

a bone-contact end surface in the form of substantially pure calcium-phosphate ceramic material, said bone-contact surface being integral with said core and having a porosity of between about 10 and about 60 percent by volume, said bone-contact surface having a thickness between about 0.25 and about 4 mm.

2. A bone graft according to Claim 1 wherein said ceramic material is of at least about 97 percent pure calcium-phosphate.

3. A graft according to Claim 1 wherein said calcium-phosphate ceramic material is selected from the group consisting of hydroxylapatite, whitlockite and mixtures thereof.

4. A graft according to Claim 1 wherein said core is hydroxylapatite and said bone-contact surface is whitlockite.

5. A graft according to Claim 1 for implantation as a bone spacer comprising a core having two spaced-apart porous bone-contact end surfaces.

6. A graft according to Claim 5 wherein the maximum cross-sectional dimension of said graft is about 40 mm and the thickness of said core between said spaced-apart end surfaces is between about 2.5 and about 40 mm.

-2-

7.    A method of forming a ceramic bone graft or the like comprising

providing a mold cavity having generally the desired shape of a bone graft,

depositing in said mold cavity a core layer and a surface layer,

said core layer comprising substantially pure calcium-phosphate ceramic material,

said surface layer comprising between about 40 to about 90 volume percent substantially pure calcium-phosphate particulate ceramic material, and between about 10 to about 60 volume percent of particulate organic material which volatilizes away within a temperature range of about 100°C and 1050°C, said surface layer being deposited at a thickness of between about 0.5 mm and about 8.0 mm,

compacting said deposited layers in said mold at pressures between about 55 and about 175MPa, and

heating said compacted layers to a temperature of between about 1050°C and about 1300°C for a period sufficient to sinter said particulate layers into a graft with a dense, non-porous core and an integral porous surface layer.

8.    A method according to Claim 7 wherein said ceramic material is at least about 97 percent calcium-phosphate ceramic material.

9.    A method according to Claim 7 wherein said calcium-phosphate ceramic material is selected from the group consisting of hydroxylapatite, whitlockite and mixtures thereof.

10.    A method according to Claim 7 wherein said dense core layer is substantially pure hydroxylapatite and said surface layer is substantially pure whitlockite.

0107476

-3-

11. A method according to Claim 7 wherein said dense core layer is substantially pure hydroxylapatite and said surface layer is a mixture of substantially pure whitlockite and substantially pure hydroxylapatite.

12. A method according to Claim 7 wherein said compacted layers are sintered for a period of from about 10 minutes to about 200 minutes.

13. A method according to Claim 7 including depositing within said mold a second surface layer of particulate organic material mixed with calcium-phosphate ceramic material.

14. A method according to Claim 7 wherein said ceramic material sifts through a screen having openings in the range of about 50-200 microns square.

15. A method according to Claim 14 wherein said powdered organic material particles sift through a screen having square openings with dimensions between two and seven times those of the screen through which said ceramic particles sift.

16. A method according to Claim 7 including machining said sintered graft.

17. A method according to Claim 7 wherein said compacted layers are sintered at a temperature of between 1050°C and 1300°C.

18. A method according to Claim 7 wherein said compacted layers are sintered at between 1100°C and 1175°C.

19. A method according to Claim 7 wherein said organic material volatilizes at a temperature of between about 100°C and about 275°C.

20. A method according to Claim 7 wherein said organic material is selected from the group consisting of PVA, PEG, urea and combinations thereof.

1/1

0107476

Fig. 1.

Fig. 2.

Fig. 3a.

Fig. 3b.

Fig. 4.

MIX → DEPOSIT LOWER SURFACE LAYER → DEPOSIT CORE LAYER → DEPOSIT UPPER SURFACE LAYER → COMPACT → MACHINE TO SHAPE / HEAT & SINTER IN KILN